# EUROPÄISCHE PATENTSCHRIFT

(11) **EP 2 529 684 B1**
(45) Veröffentlichungstag und Bekanntmachung des Hinweises auf die Patenterteilung: **02.09.2015**
(21) Anmeldenummer: 12169945.8
(22) Anmeldetag: 30.05.2012
(51) Int. Cl.: A61B 17/34

(54) **Trokarhülse**
Trocar sheath
Gaine de trocart

(30) Priorität: 31.05.2011 EP 11168177
(43) Veröffentlichungstag der Anmeldung: 05.12.2012
(73) Patentinhaber: Karl Storz GmbH & Co. KG, 78532 Tuttlingen (DE)
(72) Erfinder: Tan, Hock L. , Prof., 55000 Kuala Lumpur Selangor (MY); Oberländer, Martin, 78532 Tuttlingen (DE); Fuchs, Alexander, 78532 Tuttlingen (DE)

(56) Entgegenhaltungen:
- DE-A1-102009 014 525
- US-A1- 2002 042 606
- US-A1- 2007 162 066
- US-A1- 2008 242 930

## Beschreibung

Die Erfindung betrifft eine Trokarhülse für minimal-invasive Chirurgie, mit einem ersten Hülsenteil, der im Wesentlichen die Form eines geraden Rohrstücks mit einer Längsachse hat, und mit einem zweiten Hülsenteil, der den ersten Hülsenteil eng anliegend mindestens teilweise umgibt und im Gebrauch relativ zu dem ersten Hülsenteil beweglich ist.

Eine derartige Trokarhülse ist aus der WO 2010/136805 A1 bekannt, wobei die beiden Hülsenteile zwei teleskopartig ineinander und auseinander schiebbare gerade Rohrstücke sind, deren Relativverschiebung entweder geradlinig oder entlang einer Schraubenlinie erfolgt. Der radial äußere Hülsenteil ist an seinem proximalen Ende mit einem Kopfteil verbunden, der eine Dichtung zum gasdichten Einführen eines Instruments in die Trokarhülse und einen Fluidanschlussstutzen aufweist, und der radial innere Hülsenteil trägt an seinem distalen Ende einen flexiblen ringförmigen Flansch.

Eine Trokarhülse ist ein medizinisches Instrument, das in der minimal-invasiven Chirurgie zum Einführen von Instrumenten in den menschlichen oder tierischen Körper verwendet wird. Bei einem minimal-invasiven chirurgischen Eingriff wird ein Trokar, der aus einer Trokarhülse und einem in der Trokarhülse aufgenommenen Trokardom besteht, zunächst dazu verwendet, einen Zugang zu einer Körperhöhle zu schaffen. Dazu wird die Spitze des Trokardoms an einem Einschnitt an der Körperhaut angesetzt und dann durch die Körperdecke hindurchgestoßen. Anschließend wird der Trokardom aus der Trokarhülse herausgezogen; die Trokarhülse bleibt im Körper stecken. Durch die Trokarhülse können dann abwechselnd Instrumente wie Endoskope, Zangen, Scheren, Nahtinstrumente und dergleichen zum Durchführen chirurgischer Maßnahmen in die Körperhöhle eingeführt werden.

Der flexible Flansch am distalen Ende einer Trokarhülse entfaltet sich unterhalb der durchstoßenen Körperdecke und kann dann von außen mit einem entsprechenden weiteren Flansch gekontert werden, um für eine sichere Verankerung an der Körperdecke zu sorgen. Damit sich der Flansch unter der Körperdecke entfalten kann, muss die Trokarhülse relativ tief in den Körper hinein geschoben werden, und außerdem muss ein flexibler Flansch aus Gummi oder dergleichen relativ dick sein, um ihn wirksam von außen kontern zu können, so dass der Flansch auch während eines Eingriffs relativ viel Raum unter der Körperdecke einnimmt. Außerdem sind die meisten Trokarhülsen für eine gewisse Mindestdicke der Körperdecke ausgelegt. Aus diesen Gründen sind die bekannten Trokarhülsen für einige Operationen nicht geeignet, z. B. Operationen an Kleinkindern oder an der Schilddrüse.

Die Dokumente US 2008/0242930 A1, DE 10 2009 014 525 A1 und US 2002/0042606 A1 offenbaren jeweils ein Instrument zum Schaffen eines Zugangs für operative Eingriffe, das zwei um eine senkrecht zur Instrumentenlängsachse verlaufende Achse schwenkbare Elemente aufweist, die unterhalb der Körperdecke zu einer Art Flansch entfaltet werden können, was ebenfalls relativ viel Raum unter der Körperdecke beansprucht.

Der Erfindung liegt die Aufgabe zu Grunde, eine Trokarhülse bereitzustellen, die eine sehr geringe Einführtiefe und keine Mindestdicke der Körperdecke benötigt und die auch einfach herzustellen, leicht zu montieren und gut zu reinigen ist.

Diese Aufgabe wird durch eine Trokarhülse mit den Merkmalen des Anspruchs 1 gelöst. Vorteilhafte Weiterbildungen der Erfindung sind in den abhängigen Ansprüchen angegeben.

Gemäß der Erfindung weisen die beiden Hülsenteile an ihnen distalen axialen Enden jeweils einen Flanschteil auf, der sich über einen Winkel von weniger als 180° von dem jeweiligen Hülsenteil radial nach außen erstreckt. Wenn die Hülsenteile in eine Relativposition gedreht werden, in der die beiden Flanschteile in dieselbe Richtung weisen und im Wesentlichen deckungsgleich übereinander liegen, können die beiden Flanschteile gemeinsam durch und unter die Körperdecke geschoben werden, ohne dass sie flexibel sein müssen, weil die Elastizität der Körperdecke selbst ausreicht. Dies gilt besonders dann, wenn die Flanschteile, wie bevorzugt, ungefähr U-förmige Umrisse haben, wie in einer Ebene senkrecht zu der Längsachse gesehen, wobei der Abstand der beiden Schenkel der U-Form ungefähr gleich dem Durchmesser des ersten oder vorzugsweise des zweiten Hülsenteils ist.

Nach dem Einführen können die Hülsenteile von Hand leicht in eine Relativposition gedreht werden, in der die beiden Flanschteile in einander entgegengesetzte Richtungen weisen und in einer Ebene senkrecht zu der Längsachse liegen, so dass die Trokarhülse an der durchstoßenen Körperdecke verankert ist.

Da die Flanschteile nicht flexibel sein müssen, können sie aus einem ebenso stabilen Material wie die Hülsenteile bestehen und sind vorzugsweise damit einstückig. Derartige Flanschteile können wesentlich dünner sein als ein flexibler Flansch, beispielsweise ungefähr 1 mm dick. In der Einführposition, in der die beiden Flanschteile direkt übereinander liegen, beträgt deren Gesamtdicke dann 2 mm, was immer noch vergleichsweise wenig ist, so dass die Trokarhülse eine sehr geringe Einführtiefe in einen Körper hat.

Gemäß der Erfindung besteht die Beweglichkeit des zweiten Hülsenteils relativ zu dem ersten Hülsenteil im Wesentlichen nur in einer Drehbarkeit des zweiten Hülsenteils um den ersten Hülsenteil herum. Im Hinblick auf das Vorangehende ist dies so zu verstehen, dass eine axiale Beweglichkeit, die nur dazu dient, den einen der beiden Flanschteile, die in der Einführposition übereinander liegen, im Verlauf der Drehung in die Arbeitsposition genau in dieselbe Ebene wie jene des anderen Flanschteils zu bringen, um eine plane Verankerungsfläche unter der Körperdecke zu erzeugen, unschädlich ist. In dem genannten Beispiel von 1 mm dicken Flanschteilen würde eine derartige Verschiebung 1 mm betragen. Ebenfalls unschädlich ist es, wenn es im Verlauf einer Drehung zwischen der Einführposition und der Arbeitsposition eine besondere Montageposition gibt, in der der zweite Hülsenteil auch axial verschoben werden kann, um ihn leicht von dem ersten Hülsenteil und dem Kopfteil lösen zu können. Es ist zweckmäßig, eine derartige Montageposition vorzusehen, um die Trokarhülse leicht zusammenzubauen und für eine Reinigung schnell wieder auseinander nehmen zu können.

Prinzipiell kann jeder der beiden Hülsenteile im Wesentlichen, d. h. als einen Grundkörper, ein gerades Voll-Rohr aufweisen. In diesem Fall muss der erste, innere Hülsenteil lösbar am Kopfteil befestigt sein, beispielsweise mittels Überwurfmutter und Positionsstiften, damit der zweite Hülsenteil montiert und demontiert werden kann.

In einer alternativen Ausführungsform hat der am ersten Hülsenteil anliegende Abschnitt des zweiten Hülsenteils im Wesentlichen die Form einer Hälfte eines der Länge nach zweigeteilten Rohres. In diesem Fall kann der zweite Hülsenteil einfach axial über den ersten Hülsenteil und entlang der Längsachse in Richtung auf den Kopfteil der Trokarhülse geschoben werden und dann in die im Kopfteil ausgebildeten Führungs- und Verriegelungsmittel eingreifen gelassen werden.

Der Querschnitt des zweiten Hülsenteils senkrecht zu der Längsachse kann z. B. einfach ein Halbkreis sein.

Besser ist es, wenn der Querschnitt des zweiten Hülsenteils senkrecht zu der Längsachse ein Kreisbogen ist, der einige Winkelgrade mehr als ein Halbkreis hat. In diesem Fall umgibt der zweite Hülsenteil den ersten Hülsenteil über einen Winkel von etwas mehr als 180° und wird dadurch entlang seiner ganzen Länge formschlüssig am ersten Hülsenteil festgehalten, was auch für einen guten Zusammenhalt der beiden Hülsenteile im Gebrauch sorgt. Um das axiale Aufschieben des zweiten Hülsenteils auf den ersten Hülsenteil zu erleichtern, kann der Flanschteil des ersten Hülsenteils dort, wo er in den ersten Hülsenteil übergeht, mit kleinen Einkerbungen versehen sein.

Optimale Gasdichtigkeit zwischen Trokarhülse und der damit offen gehaltenen Körperöffnung erzielt man mit einer Ausführungsform, in der jeder der beiden Hülsenteile im Wesentlichen die Form eines geraden Voll-Rohres hat, wobei der erste, innere Hülsenteil lösbar am Kopfteil befestigt ist und der zweite Hülsenteil am ersten Hülsenteil montiert und davon demontiert werden kann, wenn der erste Hülsenteil vom Kopfteil gelöst ist.

In diesem Fall ist der zweite Hülsenteil vorzugsweise derart geführt, dass er zwischen zwei Endstellungen drehbar ist, die bezüglich der Längsachse um ungefähr 180° auseinander liegen, wobei die eine Endstellung einer Einführposition entspricht, in der die Flanschteile des ersten und des zweiten Hülsenteils in dieselbe Richtung weisen und genau in einer Ebene senkrecht zu der Längsachse liegen, und wobei die andere Endstellung einer Arbeitsposition entspricht, in der die Flanschteile des ersten und des zweiten Hülsenteils ungefähr in einander entgegengesetzte Richtungen weisen und ebenfalls genau in einer Ebene senkrecht zu der Längsachse liegen. Dabei haben die Flanschteile des ersten und des zweiten Hülsenteils in der Einführposition vorzugsweise zusammen einen U-förmigen radialen Umriss, wie in einer Ebene senkrecht zu der Längsachse gesehen.

Die lösbare Befestigung des ersten Hülsenteils am Kopfteil kann einen Schraubverschluss oder eine Art Bajonettverschluss umfassen.

Ein für die Erfindung besonders geeigneter Trokardom hat ein stumpfes distales Ende, dessen Kontur mit der Kontur des distalen Endes der Trokarhülse im Wesentlichen bündig abschließt, wenn der Trokardom vollständig in die Trokarhülse eingeführt ist.

Zum Kontern der Flanschteile auf der Außenseite der Körperdecke kann man grundsätzlich irgendein scheibenförmiges Element verwenden, insbesondere ein zweiteiliges Element, das nach der Montage des zweiten Hülsenteils rings um die Hülsenteile montiert werden kann.

Für die Erfindung besonders zweckmäßig ist jedoch ein Konterelement in Form einer einstückigen geschlitzten Gummischeibe, die bei geweitetem Schlitz von der Seite über die Hülsenteile der Trokarhülse geschoben werden kann und sich dann aufgrund ihrer Eigenelastizität mehr oder weniger fest um die Hülsenteile schließt. Nachdem man die Trokarhülse in der Körperdecke verankert hat, schiebt man die Gummischeibe einfach entlang der Längsachse in Richtung auf die Körperdecke, um die Flanschteile von außerhalb der Körperdecke zu kontern. Die Gummischeibe bleibt dann einfach durch Haftreibung in dieser Position.

Noch zweckmäßiger, weil besonders zuverlässig auf den Hülsenteilen festhaltend, ist ein Konterelement aus einem starren Element mit einem zum Außendurchmesser des äußeren Hülsenteils passenden Schlitz und einem von dem starren Element lösbaren elastischen Element, oder alternativ nur aus starren Elementen, nämlich einer Platte mit einem zum Außendurchmesser des äußeren Hülsenteils passenden Schlitz, einem auf der Platte verschiebbaren Klemmelement für den äußeren Hülsenteil und einem auf der Platte gelagerten Betätigungselement für das verschiebbare Klemmelement. Derartige Konterelemente sind auch für andere Trokarhülsen und Trokare wie hierin beschrieben nützlich.

Indem man die Trokarhülse und die Zusatzteile, die einen Trokar bilden, leicht und schnell in ihre Bestandteile zerlegen kann, kann man diese auch leicht reinigen, so dass die Trokarhülse bzw. der damit aufgebaute Trokar hohen Hygieneanforderungen genügt.

Es folgt eine Beschreibung von Ausführungsbeispielen anhand der Figuren. Es zeigen:
- Figur 1: eine Perspektivansicht einer Trokarhülse, deren äußerer Hülsenteil davon getrennt und in einer Position vor dem Zusammenbau ist;
- Figur 2: eine Perspektivansicht der Trokarhülse von Figur 1 in einer Montageposition, d. h. in einer Phase kurz bevor sie ganz zusammengebaut ist;
- Figur 3: eine Perspektivansicht der zusammengebauten Trokarhülse mit dem äußeren Hülsenteil in einer Einführposition, in der die Trokarhülse in eine in einer Körperdecke hergestellte Öffnung eingeführt werden kann;
- Figur 4: eine Perspektivansicht der zusammengebauten Trokarhülse mit dem äußeren Hülsenteil in einer Arbeitsposition, in der die Trokarhülse hinter einer in einer Körperdecke hergestellten Öffnung verankert sein kann;
- Figur 5: eine Perspektivansicht der Trokarhülse von Figur 4 mit einer aufgesteckten Gummischeibe als Konterelement;
- Figur 6: eine Seitenansicht der Trokarhülse von Figur 5, wie sie in einer Körperdecke verankert und gekontert ist;
- Figur 7: eine Perspektivansicht eines Ausführungsbeispiels für eine Trokarhülse, bei der jeder der beiden Hülsenteile im Wesentlichen die Form eines geraden Voll-Rohres hat, in einen zusammengebauten Zustand, wobei der äußere Hülsenteil in einer Einführposition ist, in der die Trokarhülse in eine in einer Körperdecke hergestellte Öffnung eingeführt werden kann;
- Figur 8: eine andere Perspektivansicht der Trokarhülse von Figur 7, an der zusätzlich ein Konterelement montiert ist;
- Figur 9: eine vergrößerte Perspektivansicht des distalen Endes der Trokarhülse von Figur 7 in der Einführposition;
- Figur 10: eine vergrößerte Perspektivansicht des distalen Endes der Trokarhülse von Figur 7 in einer Arbeitsposition, in der die Trokarhülse hinter einer in einer Körperdecke hergestellten Öffnung verankert sein kann;
- Figur 11: eine vergrößerte Perspektivansicht des distalen Endes des inneren Hülsenteils der Trokarhülse von Figur 7;
- Figur 12: eine vergrößerte Perspektivansicht des distalen Endes des äußeren Hülsenteils der Trokarhülse von Figur 7;
- Figur 13: eine vergrößerte Perspektivansicht des distalen Endes der Trokarhülse von Figur 7 im Verlauf einer Drehung des äußeren Hülsenteil von der Einführposition in die Arbeitsposition;
- Figur 14: eine unterbrochene Längsschnittansicht der beiden Hülsenteile der Trokarhülse in der Position der Figuren 7 und 9;
- Figur 15: eine Explosions-Perspektivansicht der Trokarhülse von Figur 7 im Bereich der Befestigung des inneren Hülsenteils am Kopfteil;
- Figur 16: eine vergrößerte Perspektivansicht der Überwurfmutter in Figur 15;
- Figur 17: eine vergrößerte Perspektivansicht des Federpakets in Figur 15;
- Figur 18: eine Längsschnittansicht der Trokarhülse von Figur 7 im Bereich der Befestigung des inneren Hülsenteils am Kopfteil;
- Figur 19: eine Seitenansicht einer proximalen Kulissenbahn in einem inneren Hülsenteil für eine Befestigung am Kopfteil mittels einer Art Bajonettverschluss;
- Figur 20: eine Abwicklung der Kulissenbahn des inneren Hülsenteils von Figur 19 in die Ebene;
- Figur 21: eine Schnittansicht des inneren Hülsenteils von Figur 19 und eines dazu passenden Kopfteils, die durch eine Art Bajonettverschluss miteinander verbunden sind;
- Figur 22: eine Perspektivansicht des in Figur 18 gezeigten Trokarhülsenabschnitts;
- Figur 23: eine Perspektivansicht eines Trokardoms zum Einführen in die gezeigte Trokarhülse;
- Figur 24: ein nur teilweise aus einem elastischen Material bestehendes Konterelement in einer Perspektivansicht;
- Figur 25: ein anderes nur teilweise aus einem elastischen Material bestehendes Konterelement in einer Perspektivansicht; und
- Figur 26: ein nur aus starren Teilen bestehendes Konterelement in einer Schnittansicht.

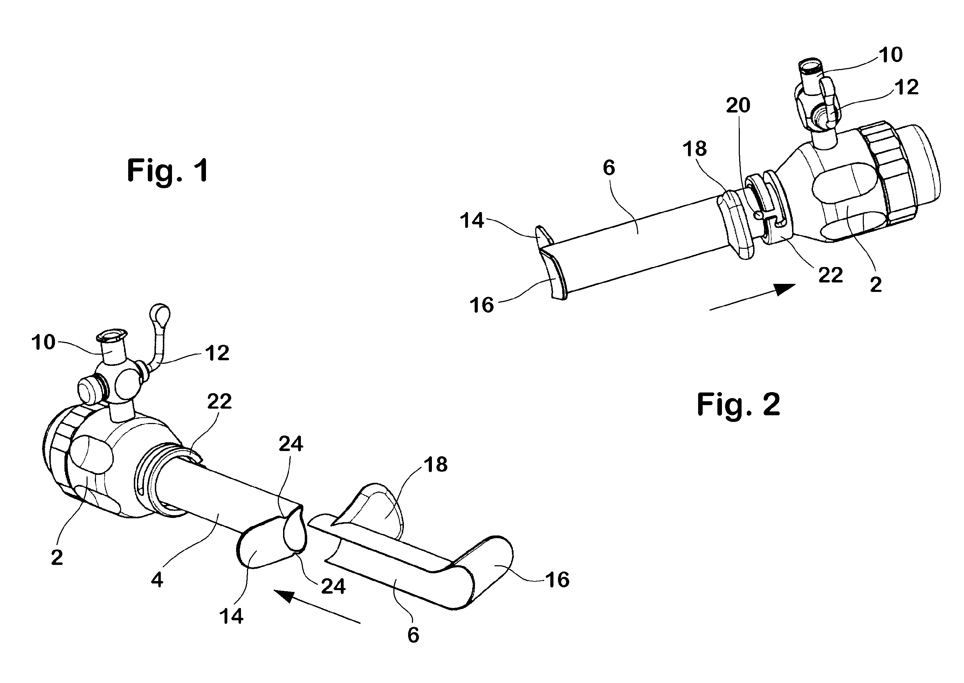

Die in Figuren 1 bis 6 gezeigte Trokarhülse besteht aus einem Kopfteil 2, einem ersten, inneren Hülsenteil 4 und einem zweiten, äußeren Hülsenteil 6.

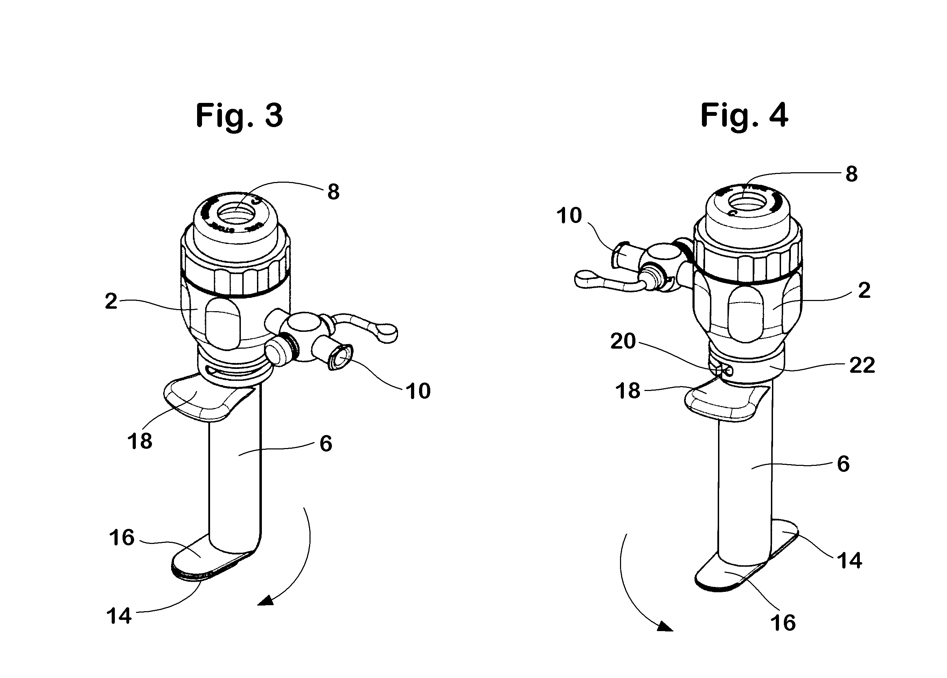

Der Kopfteil 2 ist ein ungefähr rotationssymmetrisches Gehäuse, das ein axiales Durchgangsloch enthält. In einem proximalen, in Figuren 3 bis 6 oberen Ende des Kopfteils 2 befindet sich eine flexible Dichtung 8 zum gasdichten Einführen eines Instruments in und durch das Durchgangsloch im Kopfteil 2. Außerdem hat der Kopfteil 2 einen Fluidanschlussstutzen 10 mit einem Ventil 12. Über den Fluidanschlussstutzen 10 können z. B. Kohlendioxid oder Spülflüssigkeit eingeblasen werden.

Am distalen Ende des Kopfteils 2 ist ein proximales Ende des inneren Hülsenteils 4 befestigt, welcher hauptsächlich aus einem Stück Rohr besteht, dass sich in der axialen Verlängerung des Durchgangslochs im Kopfteil 2 bis zu einem distalen Ende erstreckt.

Ganz am distalen Ende des inneren Hülsenteils 4 ist ein erster Flanschteil 14 angeformt, die ungefähr die Form eines Spatels hat. Insbesondere hat der Flanschteil 14 einen U-förmigen Umriss, wie in einer Ebene senkrecht zur Längsachse des inneren Hülsenteils 4 gesehen, wobei der Abstand der beiden Schenkel der U-Form gleich dem oder etwas kleiner als der Durchmesser des inneren Hülsenteils 4 ist. Im Ausführungsbeispiel besteht der erste Flanschteil 14 aus demselben Material wie der innere Hülsenteil 4 und ist beispielsweise 1 mm dick.

Der äußere Hülsenteil 6 besteht hauptsächlich aus einem der Länge nach halbierten Stück Rohr von derselben Länge und aus demselben Material wie der innere Hülsenteil 4. Der Querschnitt des äußeren Hülsenteils 6 senkrecht zu der Längsachse ist ein Kreisbogen, der einige Winkelgrade mehr als ein Halbkreis umfasst. Der Innendurchmesser des äußeren Hülsenteils 6 ist gleich dem Außendurchmesser des inneren Hülsenteils 4.

Am distalem Ende des äußeren Hülsenteils 6 ist ein zweiter Flanschteil 16 angeformt, der praktisch denselben Umriss und dieselbe Dicke wie der erste Flanschteil 14 hat und auf dieselbe Weise, wie der erste Flanschteil 14 einen rechtwinklig abknickenden Fortsatz des inneren Hülsenteils 4 bildet, einen radial nach außen verlaufenden und somit rechtwinklig abknickenden Fortsatz des äußeren Hülsenteils 6 bildet.

In der Nähe des proximalen Endes des äußeren Hülsenteils 6 ist ein Griffteil 18 daran angeformt, der ähnlich wie der zweite Flanschteil 16 einen radial nach außen verlaufenden Fortsatz des äußeren Hülsenteils 6 bildet, aber dicker und breiter ist. Noch etwas näher am proximalen Endes des äußeren Hülsenteils 6 ist ein kleiner runder Zapfen 20 daran angeformt, der sich vom äußeren Hülsenteil 6 einige Millimeter radial nach außen erstreckt.

Der Kopfteil 2 besitzt an seinem distalen Ende einen geschlitzten Ring 22, der sich in einem Abstand, der etwas größer als die Dicke des äußeren Hülsenteils 6 ist, radial um den inneren Hülsenteil 4 herum erstreckt. Im Zusammenwirken mit dem Zapfen 20 am äußeren Hülsenteil 6 bildet der geschlitzte Ring 22 Führungs- und Verriegelungsmittel für den äu-βeren Hülsenteil 6, und zwar nach Art eines Bajonettverschlusses, wie nachfolgend näher erläutert wird.

Wie in Figuren 1 und 2 veranschaulicht, wird die Trokarhülse montiert, indem der äußere Hülsenteil 6 in der eingezeichneten Pfeilrichtung auf den inneren Hülsenteil 4 aufgeschoben wird, wobei die beiden Flanschteile 14, 16 ungefähr in einander entgegengesetzte Richtungen weisen.

Um das Aufschieben zu erleichtern, hat der erste Flanschteil 14 zwei kleine Einkerbungen 24 in seinen Rändern, nämlich dort, wo er mit dem ersten Hülsenteil 4 verbunden ist. Die Einkerbungen 24 ermöglichen es auch, den äußeren Hülsenteil 6 um einige Winkelgrade zu verdrehen, wenn er auf dem inneren Hülsenteil 4 sitzt. Insbesondere kann man den äu-βeren Hülsenteil 6 in die in Figur 2 gezeigte Position drehen, in der die beiden Flanschteile 14, 16 in um ungefähr 160° versetzte Richtungen weisen, und dann den äußeren Hülsenteil 6 vollständig auf den inneren Hülsenteil 4 aufschieben.

Wenn der äußere Hülsenteil 6 vollständig auf den inneren Hülsenteil 4 aufgeschoben ist, wird der äußere Hülsenteil 6 um den inneren Hülsenteil 4 herum in die in Figur 3 gezeigte Position gedreht, in der die beiden Flanschteile 14 und 16 deckungsgleich übereinander liegen. Während der Drehung, deren Drehrichtung in Figur 3 mit einem Pfeil angezeigt ist, gleitet der Zapfen 20 in dem geschlitzten Ring 22 und wird dadurch geführt.

In der in Figur 3 gezeigten Position können die beiden Flanschteile 14 und 16 der Trokarhülse leicht in eine in einer Körperdecke hergestellte Öffnung eingeführt werden, ohne tief darin einzudringen, indem die Trokarhülse mehr oder weniger senkrecht über der Körperdecke gehalten und etwas nach unten und zur Seite bewegt wird, damit die beiden Flanschteile 14 und 16 gemeinsam unter die Körperdecke gleiten.

Nachdem die Trokarhülse ihre Endposition in und über der Körperöffnung erreicht hat, wird der äußere Hülsenteil 6 mit Hilfe des Griffteils 18 um den inneren Hülsenteil 4 herum zurückgedreht, nämlich in die in Figur 4 gezeigte Position, in der die beiden Flanschteile 14 und 16 in einander genau entgegengesetzte Richtungen weisen und genau in einer Ebene senkrecht zu der Längsachse der Hülsenteile 4 und 6 liegen. Die Drehrichtung ist in Figur 4 mit einem Pfeil angezeigt. In dieser Position wird der Zapfen 20 am ersten Hülsenteil 4 durch eine Hinterschneidung im geschlitzten Ring 22 festgehalten, so dass der zweite Hülsenteil 6 an der Trokarhülse verriegelt ist. In den durch seine Eigenelastizität gegebenen Grenzen ist der zweite Hülsenteil 6 auch entlang seiner Länge verriegelt, weil der äußere Hülsenteil 6 den inneren Hülsenteil 4 um mehr als 180° umgreift.

Die Führungswirkung des geschlitzten Rings 22 am Kopfteil ist entweder rein rotatorisch, oder sie bewirkt auch eine geringfügige Längsverschiebung von der Dicke der Flanschteile 14 und 16, wodurch der zweite Flanschteil 16 automatisch aus der in Figur 3 gezeigten Position, in der er deckungsgleich über dem ersten Flanschteil 14 liegt, in die in Figur 4 gezeigte Position versetzt wird, in der er genau in einer Ebene mit dem ersten Flanschteil 14 liegt. Ein derartiger Versatz kann aber auch durch entsprechende Ausbildung des Abschnitts des geschlitzten Rings 22 erzielt werden, in dem der Zapfen 20 durch eine Hinterschneidung im geschlitzten Ring 22 verriegelt wird.

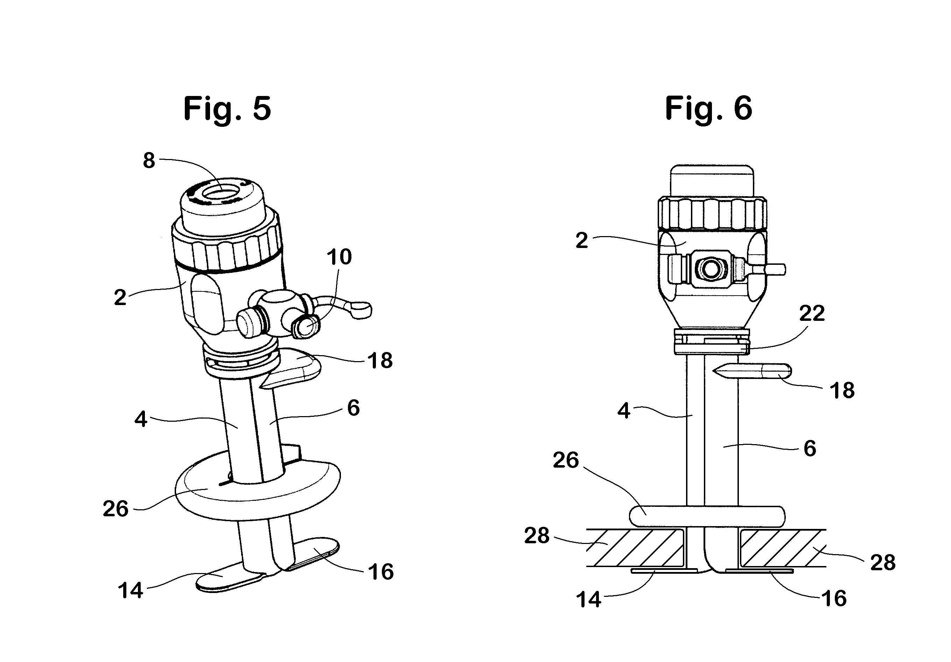

Wie in Figur 5 gezeigt, kann nach dem Verankern der Trokarhülse in einer Körperöffnung eine geschlitzte und von Hand auseinander ziehbare Gummischeibe 26, beispielsweise in Gestalt eines so genannten Optikstoppers, von der Seite über beide Hülsenteile 4 und 6 der Trokarhülse geschoben werden. Wenn sich die Gummischeibe 26 wieder zusammenzieht, umschließt sie die Hülsenteile 4 und 6 mit einer gewissen Kraft. Die Gummischeibe 26 kann dann von Hand in Richtung auf die Flanschteile 14 und 16 geschoben werden, bis eine in Figur 6 schematisch im Schnitt eingezeichnete Körperdecke 28 unten an den Flanschteilen 14, 16 und oben an der Gummischeibe 26 anliegt, so dass die Trokarhülse sicher an der Körperdecke 28 verankert ist.

Statt der Gummischeibe 26 kann man auch irgendein anderes geeignetes Konterelement verwenden, z. B. ein zweiteiliges Konterelement, das um die Hülsenteile 4 und 6 herum montiert werden kann.

In dem gezeigten Ausführungsbeispiel sind die Flanschteile 14 und 16 spatelförmig bzw. U-förmig. Obwohl diese spezielle Ausfiihrungsform besonders vorteilhaft ist, was ein gewebeschonendes und nicht zu tiefes Einführen in die Körperdecke angeht, sind auch andere Umrissgestalten möglich. Beispielsweise können die Flanschteile 14 und 16 irgendeine Blattform haben, wie sie bei Pflanzenblättern vorkommt, doch sollten ihre Umrisse gerundet sein. Wesentlich ist, dass sich jeder Flanschteil 14, 16 über einen Winkel von weniger als 180° von dem jeweiligen Hülsenteil 4 bzw. 6 radial nach außen erstreckt.

In dem gezeigten Ausführungsbeispiel sind die Flanschteile 14 und 16 außerdem eben und liegen stets parallel zueinander. Sie könnten z. B. aber auch Kegelabschnitte oder Kugelabschnitte ausbilden, was für Operationen an eher konvexen oder konkaven Körperteilen vorteilhaft sein kann.

Ein weiteres Ausführungsbeispiel unterscheidet sich von dem Ausführungsbeispiel der Figuren 1 bis 6 im Wesentlichen darin, dass der Grundkörper des äußeren Hülsenteils keinen halbkreisförmigen Querschnitt hat, wie bei dem äußeren Hülsenteil 6, sondern ebenso wie bei dem inneren Hülsenteil 4 ein Voll-Rohr ist. Um in diesem Fall den äußeren Hülsenteil montieren und demontieren zu können, muss das distale Ende des inneren Hülsenteils lösbar am Kopfteil befestigt sein, z. B. mittels Überwurfmutter und Positionsstiften.

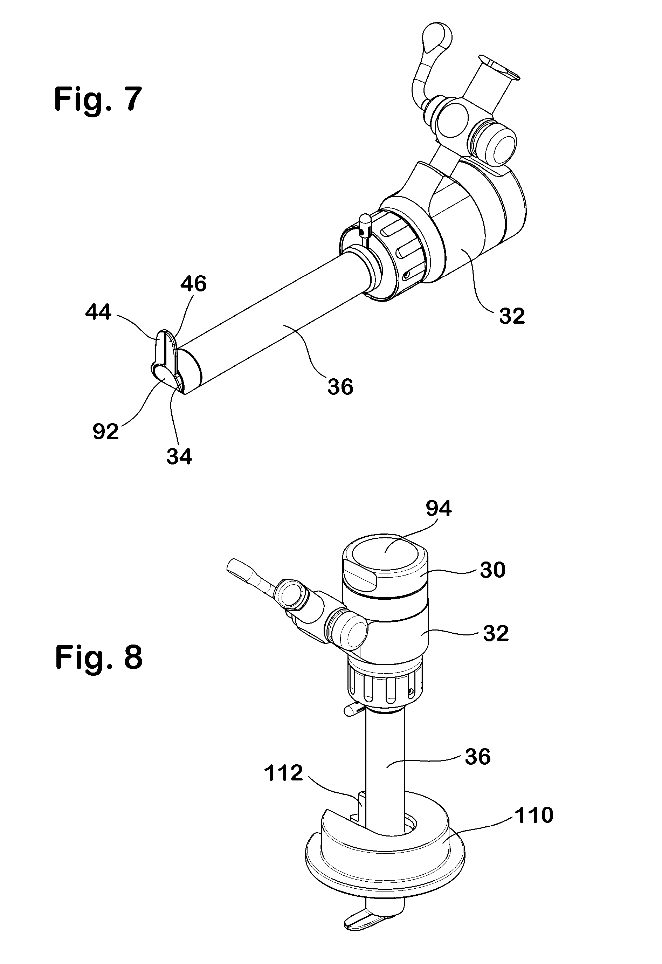

Dieses Ausführungsbeispiel, das sich auch in weiteren Details von dem Ausführungsbeispiel der Figuren 1 bis 6 unterscheidet, wird nachfolgend anhand der Figuren 7 bis 26 näher beschrieben.

Die Trokarhülse mit zwei Voll-Rohren als Hülsenteile enthält einen Kopfteil 32, einen ersten, inneren Hülsenteil 34 und einen zweiten, äußeren Hülsenteil 36. Der Kopfteil 32 ist dem Kopfteil 2 des vorhergehenden Ausführungsbeispiels ähnlich, außer dass der innere Hülsenteil 34, welcher hauptsächlich aus einem Stück Rohr besteht, das sich in der axialen Verlängerung des Durchgangslochs im Kopfteil 32 bis zu einem distalen Ende erstreckt, lösbar am distalen Ende des Kopfteils 2 montiert ist. Der äußere Hülsenteil 36 ist ebenso wie der innere Hülsenteil 34 als Voll-Rohr ausgeführt, und der innere Hülsenteil 34 kann beginnend mit seinem distalen Ende in den äußeren Hülsenteil 36 hineingeschoben werden, wobei der äußere Hülsenteil 36 den inneren Hülsenteil 34 mit geringem Spiel umschließt.

Am distalen Ende des inneren Hülsenteils 34 ist ein erster Flanschteil 44 angeformt, der ungefähr die Form eines der Länge nach halbierten Spatels hat und einen radial nach außen verlaufenden und somit rechtwinklig abknickenden Fortsatz des inneren Hülsenteils 34 bildet. Am distalem Ende des äußeren Hülsenteils 36 ist ein zweiter Flanschteil 46 angeformt, der die Form der anderen Hälfte des der Länge nach halbierten Spatels hat und einen radial nach außen verlaufenden und somit rechtwinklig abknickenden Fortsatz des äußeren Hülsenteils 36 bildet.

Wenn der innere Hülsenteil 34 und der äußere Hülsenteil 36 vollständig der Länge nach zusammengeschoben sind, wie in Figuren 7 bis 10, 13 und 14 gezeigt, erstrecken sich die beiden Flanschteile 44 und 46 genau in einer Ebene senkrecht zu der Längsachse der Hülsenteile 34 und 36.

In der in Figuren 7, 8, 9 und 14 gezeigten Einführposition ergänzen sich die beiden Flanschteile 44 und 46 außerdem zu der Form des (halbierten) Spatels, indem sie zusammen einen U-förmigen Umriss haben, wie in einer Ebene senkrecht zur Längsachse der Hülsenteile 34 und 36 gesehen, wobei der Abstand der beiden Schenkel der U-Form ungefähr gleich dem oder etwas kleiner als der Durchmesser des inneren Hülsenteils 34 ist.

Die dem Flanschteil 44 entgegengesetzte Seite des inneren Hülsenteils 34 besitzt eine vorstehende Nase 38, die in eine zum distalen Ende hin offene Nut 40 passt, die in der dem Flanschteil 44 entgegengesetzten Seite des äußeren Hülsenteils 36 ausgebildet ist. Die Nut 40 geht in eine radial umlaufende Rille 42 im Innenumfang des äußeren Hülsenteils 36 über.

In der in Figuren 7 bis 10, 13 und 14 gezeigten Winkel-Relativposition können der innere Hülsenteil 34 und der äußere Hülsenteil 36 vollständig der Länge nach zusammengeschoben werden, wobei die Nase 38 in die Nut 40 rutscht und an deren Boden anschlägt. Zugleich schlägt die nach proximal weisende Planfläche am Flanschteil 44 des inneren Hülsenteils 34 an einer distalen Stirnseite des äußeren Hülsenteils 36 an. Die beiden Flanschteile 44 und 46 liegen dann in derselben Ebene senkrecht zur Längsachse der Hülsenteile 34 und 36.

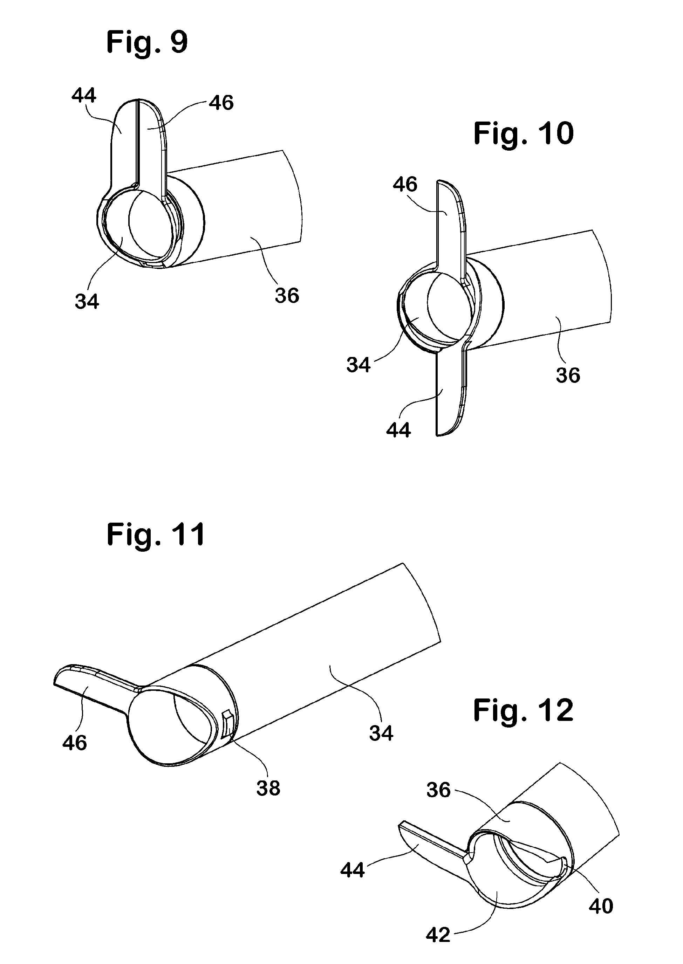

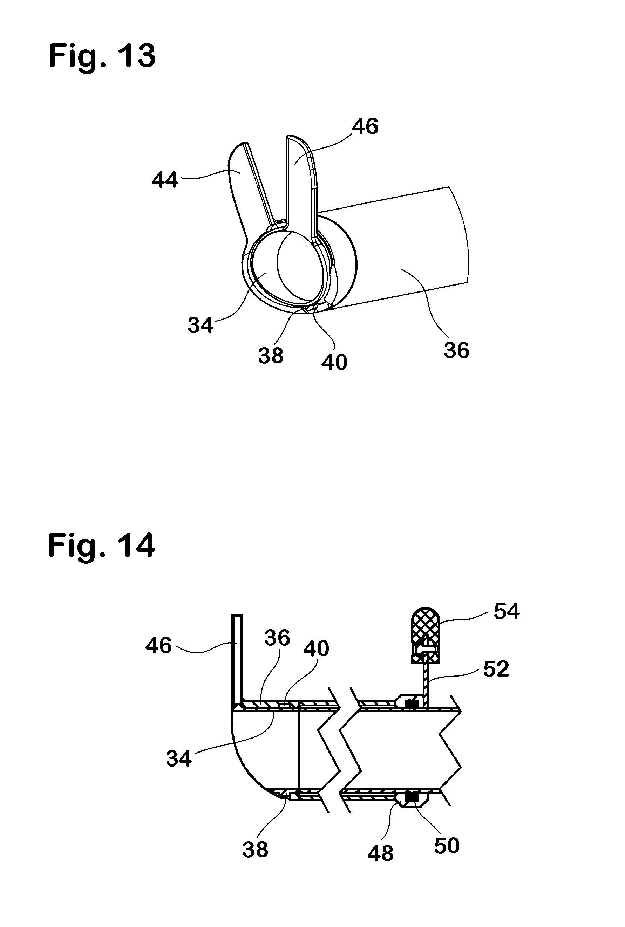

Wird nun der äußere Hülsenteil 36 etwas um den inneren Hülsenteil 34 gedreht, wie in Figur 13 gezeigt, tritt die Nase 38 am inneren Hülsenteil 34 in die Rille 42 im äußeren Hülsenteil 36 ein, so dass der innere Hülsenteil 34 und der äußere Hülsenteil 36 nicht mehr axial zueinander verschoben werden können. Der äußere Hülsenteil 36 kann dann weiter um den inneren Hülsenteil 34 gedreht werden, bis sich die beiden Flanschteile 44 und 46 in einer Arbeitsposition oder OP-Position befinden, in der sie ungefähr in einander entgegengesetzte Richtungen weisen, wie in Figur 10 gezeigt, um die Trokarhülse unterhalb einer Körperdecke zu verankern. Während des ganzen Drehvorgangs bleiben die beiden Flanschteile 44 und 46 in derselben Ebene senkrecht zur Hülsenlängsachse.

Wenn der innere Hülsenteil 34 am Kopfteil 32 befestigt ist, wird in der Einführposition, in der die Nase 38 noch nicht in die Rille 42 eingreift, eine axiale Verschiebung des äußeren Hülsenteils 36 in proximale Richtung durch den Kopfteil 32 begrenzt.

Wie in Figur 14 gezeigt, befindet sich am proximalen Ende des äußeren Hülsenteils 36 eine radial umlaufende Manschette 48, in die ein O-Ring 50 passt, der den äußeren Hülsenteil 36 gegen den längeren inneren Hülsenteil 34 abdichtet. An der Manschette 48 ist außerdem ein Betätigungshebel zum Drehen des äußeren Hülsenteil 36 befestigt. Der Betätigungshebel besteht aus einem in Hülsenlängsrichtung einfedernden Federblech 52 mit runder Kunststoffkappe 54, deren Funktion weiter unten erläutert wird.

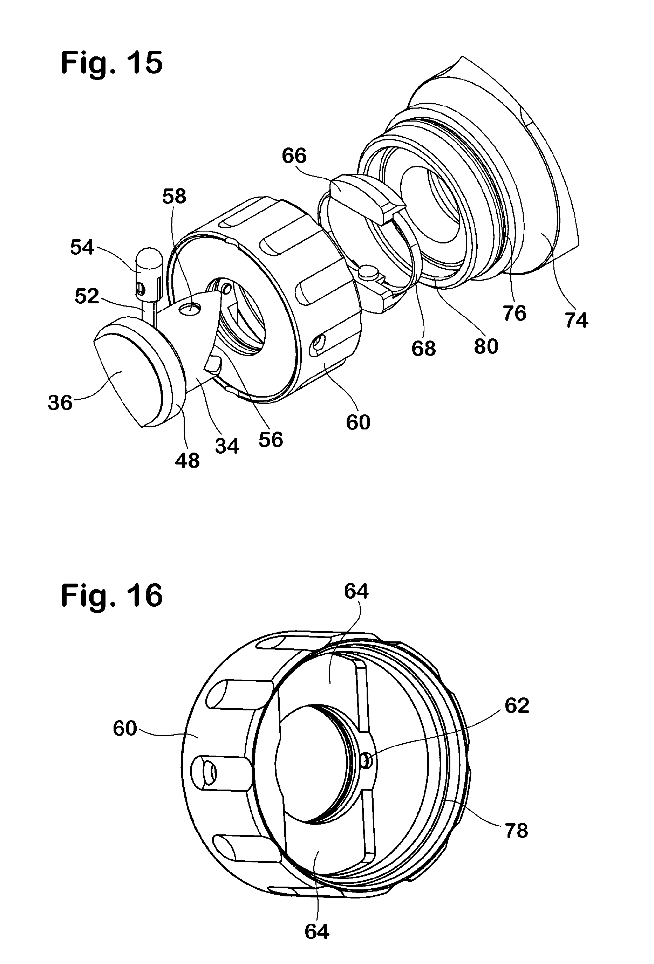

Für die Befestigung des inneren Hülsenteils 34, an dem der äußere Hülsenteil 36 angebracht ist, am Kopfteil 32 gibt es mehrere Möglichkeiten. Ein Beispiel für eine Befestigung des inneren Hülsenteils 34 am Kopfteil 32 mittels Schraubverschluss wird unter Bezugnahme auf Figuren 15 bis 18 beschrieben.

In Figur 15 erkennt man das proximale Ende des inneren Hülsenteils 34 mit dem daran montierten äußeren Hülsenteil 36. Das proximale Ende des inneren Hülsenteils 34 hat eine Doppel-V-förmige Kontur 56 mit zwei Arretierungslöchern 58 in den Spitzen der Doppel-V-förmigen Kontur 56. Eine Überwurfmutter 60, in deren kleinsten Durchmesser der innere Hülsenteil 34 gerade hineinpasst, besitzt auf ihrer Innenseite zwei Stifte 62 (Figur 16), die über den kleinsten Durchmesser hinaus radial nach innen vorstehen. Die Täler der Doppel-V-förmigen Kontur 56 sind mit demselben Radius wie die Stifte 62 verrundet.

Wird das proximale Ende des inneren Hülsenteils 34 an der Seite mit dem kleinsten Durchmesser in die Überwurfmutter 60 geschoben, dann berühren die Stifte 62 der Überwurfmutter 60 die schrägen Seiten der Doppel-V-förmigen Kontur 56. Wird der innere Hülsenteil 34 weiter in die Überwurfmutter 60 geschoben, wird dadurch die Überwurfmutter 60 gedreht, bis die Stifte 62 genau in den Tälern der Doppel-V-förmigen Kontur 56 liegen. Dadurch nimmt der innere Hülsenteil 34 sowohl radial als auch axial eine feste Position in der Überwurfmutter 60 an.

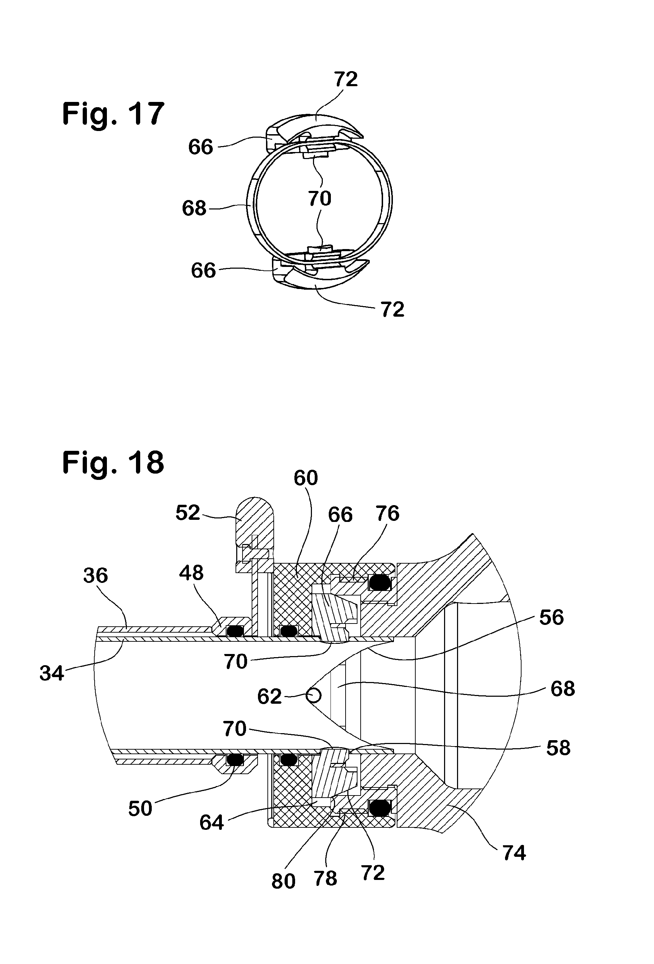

Im Inneren der Überwurfmutter 60 befinden sich zwei Ausnehmungen 64, die sich senkrecht zur Achse der Stifte 62 erstrecken. In die Ausnehmungen 64 passt je ein Abschnitt von einem von zwei Nutensteinen 66, die an einander entgegengesetzten Stellen einer ovalen Feder 68 befestigt sind, um ein Federpaket zu bilden, wie es in Figur 17 gezeigt ist. Die ovale Feder 68 übt eine Vorspannkraft radial nach außen auf die Nutensteine 66 aus, wenn das Federpaket in der Überwurfmutter 60 sitzt, wie in Figur 18 gezeigt.

Die Nutensteine 66 tragen je einen Bolzenfortsatz 70, der genau in ein Arretierungsloch 56 des inneren Hülsenteils 34 passt und damit fluchtet, wenn die Stifte 62 der Überwurfmutter 60 genau in den Tälern der Doppel-V-förmigen Kontur des inneren Hülsenteils 34 liegen. An den Nutensteinen 66 sind außerdem nach schräg außen weisende konische Flächen 72 ausgebildet.

Der in Figuren 15 und 18 gezeigte Einschraubabschnitt 74 des Kopfteils 32 trägt ein Au-βengewinde 76, das zu einem Innengewinde 78 in der Überwurfmutter 60 passt. Dreht man den Einschraubabschnitt 74 in die Überwurfmutter 60 ein, während sich der innere Hülsenteil 34 und das Federpaket darin befinden, dann drückt ein Kegelabschnitt 80 am Einschraubabschnitt 74 gegen die konischen Flächen 72 der Nutensteine 66. Dadurch werden die Nutensteine 66 gegen die Federkraft der ovalen Feder 68 nach innen bewegt, wodurch die Bolzenfortsätze 70 in die Arretierungslöcher 56 im inneren Hülsenteil 34 eingreifen und somit den inneren Hülsenteil 34 am Kopfteil 32 arretieren.

Beim Lösen der Gewinde 76 und 78 voneinander bewegen sich die Nutensteine 66 nach außen, so dass die Bolzenfortsätze aus den Arretierungslöchern 56 austreten, und der innere Hülsenteil 34 kann zusammen mit dem äußeren Hülsenteil 36 aus dem Kopfteil 32 herausgezogen werden. Anschließend können die Hülsenteile 34 und 36 voneinander getrennt werden.

Wie man in Figur 18 erkennt, dichten zwei O-Ringe die Überwurfmutter 60 gegen den inneren Hülsenteil 34 bzw. gegen den Einschraubabschnitt 74 des Kopfteils 32 ab.

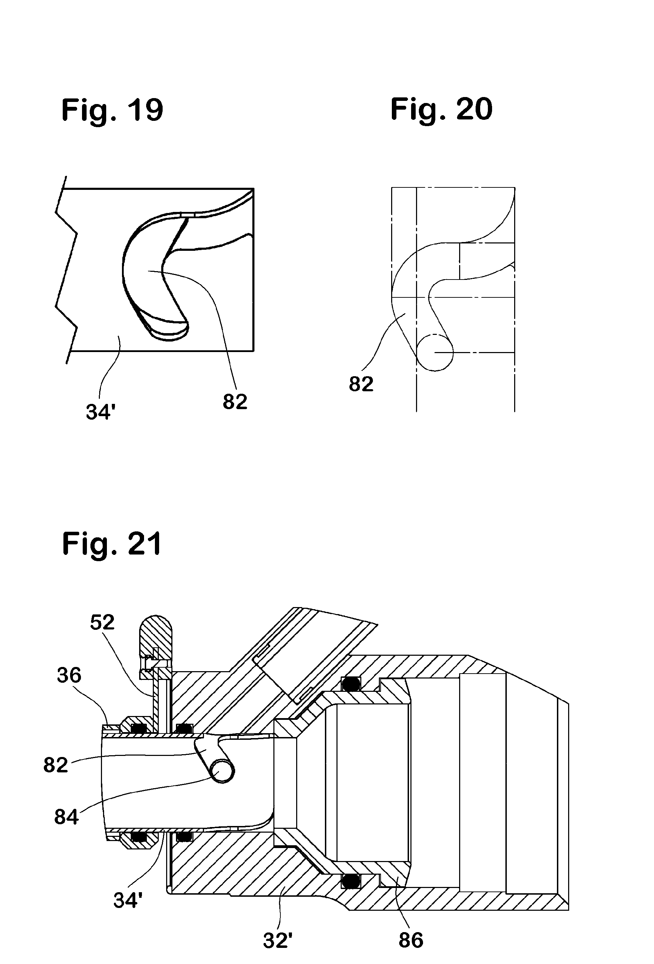

Unter Bezugnahme auf Figuren 19 bis 21 wird nun ein Beispiel für eine Befestigung eines inneren Hülsenteils 34' an einem Kopfteil 32' mittels einer Art Bajonettverschluss beschrieben, der sich von einem gewöhnlichen Bajonettverschluss jedoch darin unterscheidet, dass der Verschluss nicht durch einfaches Drehen der verbundenen Teile relativ zueinander gelöst werden kann.

Der innere Hülsenteil 34' und der Kopfteil 32' unterscheiden sich nur im Bereich der Befestigung miteinander von dem vorher beschriebenen inneren Hülsenteil 34 und Kopfteil 32. Und zwar ist im Bereich des proximalen Endes des inneren Hülsenteils 34' eine Kulissenbahn 82 in deren Außenumfang ausgespart, die von der Seite betrachtet wie in Figur 19 aussieht und in eine Ebene abgewickelt wie in Figur 20 aussieht. Vom Innenumfang des Kopfteils 32' steht ein zur Kulissenbahn 82 passender Bajonettstift 84 (Figur 21) radial nach innen vor.

Zur Befestigung des inneren Hülsenteils 34' am Kopfteil 32' wird der Kopfteil 32' derart auf den inneren Hülsenteil 34' aufgeschoben, dass der Bajonettstift 84 in die Kulissenbahn 82 eingreift und nachfolgend davon geführt wird. Wird der Kopfteil 32' nachfolgend weiter auf den inneren Hülsenteil 34' geschoben und gedreht, dann bewegt sich der Kopfteil 32' zunächst weiter nach distal über den inneren Hülsenteil 34', bis er am distal weitesten Punkt der Kulissenbahn 82 anschlägt. Dreht man nun den Kopfteil 32' weiter, dann bewegt er sich wieder ein kleines Stück weit in proximale Richtung. In dieser Position wird der innere Hülsenteil 34' am Kopfteil 32' fixiert, indem vom proximalen Ende des Kopfteils 32' her eine Abstandshülse 86 in den Kopfteil 32' eingeführt wird, bis sie am inneren Hülsenteil 34' anschlägt. Dies ist möglich, weil die Kopfteile 32 und 32' jeweils einen abschraubbaren Deckel 30 (siehe Figur 8) am proximalen Ende aufweisen. Indem man den Deckel 30 nach dem Einlegen einer Dichtung in den Kopfteil 32' wieder auf den Kopfteil 32' aufschraubt, wird die Abstandshülse 86 und damit der innere Hülsenteil 34' am Kopfteil 32' fixiert. Da sich in diesem Zustand axiale und radiale Bewegungen überschneiden, ist durch die axiale Fixierung auch keine radiale Bewegung zwischen innerem Hülsenteil 34' und Kopfteil 32' möglich.

In dem Bereich zwischen maximaler axialer Relativverschiebung und axialer Relatiwerschiebung im montierten Zustand, wie in Figur 21 gezeigt, wird außerdem das Federblech 52 des Betätigungshebels am äußeren Hülsenteil 36 elastisch verformt. Dies liefert eine haptische Rückmeldung über das Einrasten des Bajonettverschlusses.

Ebenso wie im zuerst beschrieben Ausführungsbeispiel ist auch im Ausführungsbeispiel der Figuren 7 bis 21 die Trokarhülse einfach und schnell in mehrere Bestandteile zerlegbar, was deren Sterilisation erleichtert.

Merkmale des Ausführungsbeispiels der Figuren 7 bis 21 können auch mit Merkmalen des Ausführungsbeispiels der Figuren 1 bis 6 kombiniert werden.

Es folgt eine Beschreibung von weiteren Details und Zusatzteilen des Ausführungsbeispiels der Figuren 7 bis 21, die auch bei dem Ausführungsbeispiel der Figuren 1 bis 6 angewendet werden können.

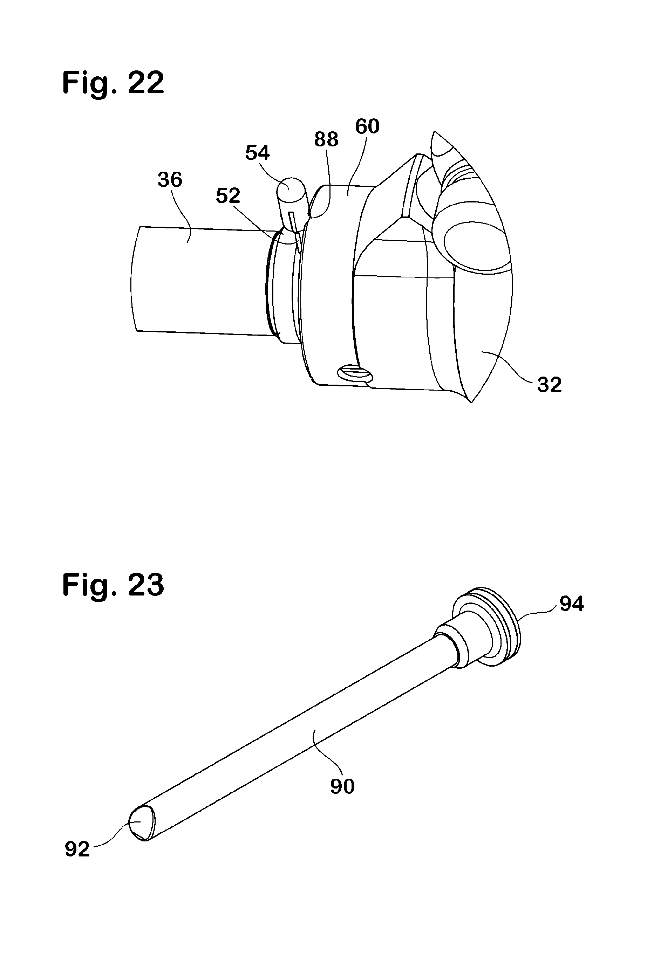

Um dem Anwender zu signalisieren, in welcher Stellung sich das Instrument befindet, d. h. in der Einführposition, in der die Flanschteile 44, 46 der beiden Hülsenteile 34, 36 in dieselbe Richtung weisen, oder in der Arbeitsposition, in der die Flanschteile 44, 46 ungefähr in einander entgegengesetzte Richtungen weisen, sind an einer distalen Planfläche des Kopfteils 32 oder 32' bzw. der Überwurfmutter 60 zwei teilzylindrische Einkerbungen vorgesehen, die um 180° zueinander versetzt sind. In Figur 22, die den in Figur 18 gezeigten Trokarhülsenabschnitt perspektivisch zeigt, sind dies zwei zylindrische Einkerbungen 88 für die Einführposition bzw. die Arbeitsposition, von denen in der Figur nur eine sichtbar ist, an der Überwurfmutter 60. Solange sich die Kunststoffkappe 54 des Betätigungshebels in einer dieser Einkerbungen 88 befindet, ist dessen Federblech 52 unbelastet. Wird der äußere Hülsenteil 36 mittels des Betätigungshebels gedreht, muss der Betätigungshebel den Rand der entsprechenden Einkerbung 88 überwinden. Damit ändert sich der axiale Abstand zwischen Kopfteil 32 und äußerem Hülsenteil 36, und das Federblech 52 wird vorgespannt. Sobald sich der Betätigungshebel wieder über eine der Einkerbungen 88 bewegt, rastet er leicht darin ein, indem sich das Federblech 52 entspannt.

Figur 23 zeigt einen Trokardom 90, der besonders geeignet ist, um die beschriebenen Trokarhülsen in einen Körper einzuführen. Der Trokardom 90 hat statt der sonst üblichen scharfen Spitze ein stumpfes distales Ende 92 mit einer leicht gekrümmten Stirnfläche mit kleinen Fasen oder Rundungen im Übergang zum zylindrischen Schaft des Trokardoms 90. Die Kontur des distalen Endes 92 des Trokardoms 90 schließt mit der Kontur des distalen Endes der Trokarhülse, welche in Figur 14 genauer zu erkennen ist, im Wesentlichen bündig ab, wenn der Trokardom 90 vollständig in die Trokarhülse eingeführt ist, wie in Figur 7 zu sehen ist. Dieser Zustand ist für einen Bediener auch daran zu erkennen, dass das proximale Ende 94 des Trokardoms 90 mit dem proximalen Ende des Trokarkopfes 32 bündig abschließt, wie in Figur 8 zu sehen ist.

Zum Gebrauch wird der Trokardom 90 von proximal durch die Trokarhülse gesteckt, um deren distales Ende damit zu schließen. Der von den Flanschteilen 44 und 46 (oder 14 und 16) geformte Spatel wird in einen Skalpell-Einschnitt eingeführt, während der Trokar schräg über der Körperdecke gehalten wird. Der Trokar wird in die Senkrechte geschwenkt, und die Hülsenteile 34 und 36 und damit die Flanschteile 44 und 46 werden mittels des Betätigungshebels gegeneinander verdreht, wodurch der Trokar atraumatisch in die Körperöffnung eingeführt wird. Anschließend kann der Trokardom 90 aus der Trokarhülse herausgezogen werden. Die Trokarhülse ist nun offen, um durch die Trokarhülse hindurch einen minimal-invasiven Eingriff durchzuführen.

Anschließend werden die Flanschteile 44 und 46 auf der Außenseite der Körperdecke mit einem Konterelement gekontert, das den äußeren Hülsenteil 36 umschließt und unter Überwindung bzw. Ausschaltung seiner Klemmkraft axial entlang des äußeren Hülsenteils 36 verschoben werden kann. Zu diesem Zweck noch besser geeignet als die vorher beschriebene einstückige geschlitzte Gummischeibe sind Konterelemente, wie sie nachfolgend beschrieben werden.

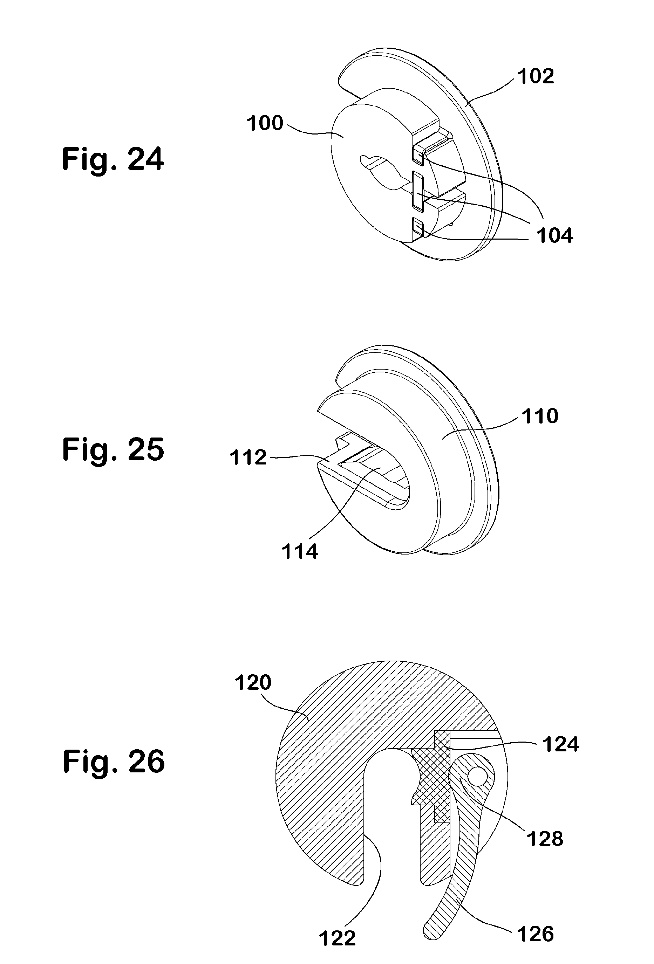

Das in Figur 24 gezeigte Konterelement ist in zwei Teile zerlegbar, nämlich in ein geschlitztes scheibenförmiges Teil 100 aus einem elastischen Material wie z. B. Silikon, das einen etwas kleineren Innendurchmesser als der Außendurchmesser des äußeren Hülsenteils 36 hat, und eine geschlitzte Auflageplatte 102 aus einem starren Material wie z. B. Stahl. Rechtwinklig von der Auflageplatte 102 stehen Fortsätze 104 in dazu passende Ausnehmungen im elastischen Teil 100 vor. Die Fortsätze 104 halten das elastische Teil 100 an der starren Auflageplatte 102 fest und stabilisieren es, um genug Klemmkraft zu erzielen. Das elastische Teil 100 und die Auflageplatte 102 sind weit genug geschlitzt, dass man sie getrennt seitlich auf den äußeren Hülsenteil 36 aufschieben und dann in Axialrichtung zusammenstecken kann.

Auch das in Figur 25 gezeigte Konterelement ist in zwei Teile zerlegbar, nämlich in eine ringförmige Umrandung 110 aus einem starren Material wie z. B. Stahl mit einem U-förmigen Schlitz 112 und eine Einlage 114 aus einem elastischen Material wie z. B. Silikon im Inneren des Schlitzes 112, die durch Form- und/oder Kraftschluss etwas im Schlitz 112 fixiert sein kann, aber herausnehmbar ist. Die Umrandung 110 stabilisiert die Einlage 114, um genug Klemmkraft zu erzielen. Die Umrandung 110 und die Einlage 114 können gemeinsam oder nacheinander auf den äußeren Hülsenteil 36 aufgeschoben und dann axial daran entlang in die gewünschte Position verschoben werden, wie in Figur 8 veranschaulicht.

Das in Figur 26 gezeigte Konterelement ist nicht zerlegbar und besteht aus einer ungefähr starren Platte 120 mit einem radialen Schlitz 122, einem in Richtung auf das innere Ende des Schlitzes 122 verschiebbaren Klemmelement 124 und einem Betätigungshebel 126, der schwenkbar an der Platte 120 gelagert ist und an seiner Basis einen Exzenter 128 aufweist, der an dem Klemmelement 124 angreift. Nachdem dieses Konterelement seitlich auf den äußeren Hülsenteil 36 aufgeschoben worden ist, wird das Klemmelement 124 durch Schwenken des Betätigungshebels 126 gegen den äußeren Hülsenteil 36 gedrückt und bleibt durch Reibung des Exzenters 128 am Klemmelement 124 in dieser Stellung.

### Bezugszeichen

- 2: Kopfteil
- 4: erster, innerer Hülsenteil
- 6: zweiter, äußerer Hülsenteil
- 8: flexible Dichtung
- 10: Fluidanschlussstutzen
- 12: Ventil
- 14: erster Flanschteil
- 16: zweiter Flanschteil
- 18: Griffteil am äußeren Hülsenteil 6
- 20: Zapfen am äußeren Hülsenteil 6
- 22: geschlitzter Ring
- 24: Einkerbungen in ersten Flanschteil 14
- 26: Gummischeibe
- 28: Körperdecke
- 32: Kopfteil
- 34; 34': erster, innerer Hülsenteil
- 36; 36': zweiter, äußerer Hülsenteil
- 38: Nase
- 40: Nut
- 42: Rille
- 44: erster Flanschteil
- 46: zweiter Flanschteil
- 48: Manschette
- 50: O-Ring
- 52: Federblech des Betätigungshebels
- 54: Kunststoffkappe des Betätigungshebels
- 56: Doppel-V-förmige Kontur
- 58: Arretierungslöcher
- 60: Überwurfmutter
- 62: Stifte
- 64: Ausnehmungen
- 66: Nutensteine des Federpakets
- 68: ovale Feder des Federpakets
- 70: Bolzenfortsätze an den Nutensteinen 66
- 72: konische Flächen an den Nutensteinen 66
- 74: Einschraubabschnitt des Kopfteils 32
- 76: Außengewinde am Kopfteil 32
- 78: Innengewinde 78 in der Überwurfmutter 60
- 80: Kegelabschnitt am Einschraubabschnitt 74
- 82: Kulissenbahn
- 84: Bajonettstift
- 86: Abstandshülse
- 88: zylindrische Einkerbungen
- 90: Trokardom
- 92: distales Ende des Trokardoms 92
- 94: proximales Ende des Trokardoms 92
- 100: elastisches Teil eines Konterelements
- 102: Auflageplatte des Konterelements
- 104: Fortsätze an der Auflageplatte 102
- 110: Umrandung eines Konterelements
- 112: U-förmiger Schlitz in der Umrandung 110
- 114: Einlage in der Umrandung 110
- 120: starre Platte eines Konterelements
- 122: radialer Schlitz in der Platte 120
- 124: Klemmelement an der Platte 120
- 126: Betätigungshebel an der Platte 120
- 128: Exzenter am Betätigungshebel 126

## Patentansprüche

1. Trokarhülse für minimal-invasive Chirurgie, mit einem ersten Hülsenteil (4; 34), der im Wesentlichen die Form eines geraden Rohrstücks mit einer Längsachse hat, und mit einem zweiten Hülsenteil (6; 36), der den ersten Hülsenteil (4) eng anliegend mindestens teilweise umgibt und im Gebrauch relativ zu dem ersten Hülsenteil (4) beweglich ist,
**dadurch gekennzeichnet,**
**dass** die Beweglichkeit des zweiten Hülsenteils (6; 36) relativ zu dem ersten Hülsenteil (4) im Wesentlichen nur in einer Drehbarkeit des zweiten Hülsenteils (6; 36) um den ersten Hülsenteil (4; 34) herum besteht und dass der erste Hülsenteil (4; 34) und der zweite Hülsenteil (6; 36) an ihren distalen axialen Enden jeweils einen Flanschteil (14, 16; 44, 46) aufweisen, der sich über einen Winkel von weniger als 180° von dem jeweiligen Hülsenteil (4, 6; 34, 36) radial nach außen erstreckt.

2. Trokarhülse nach Anspruch 1, **dadurch gekennzeichnet, dass** der zweite Hülsenteil (6) derart geführt ist, dass er zwischen zwei Endstellungen drehbar ist, die bezüglich der Längsachse um ungefähr 180° auseinander liegen, wobei die eine Endstellung einer Einführposition entspricht, in der die Flanschteile (14, 16) des ersten und des zweiten Hülsenteils (4, 6) in dieselbe Richtung weisen und im Wesentlichen übereinander liegen, und wobei die andere Endstellung einer Arbeitsposition entspricht, in der die Flanschteile (14, 16) des ersten und des zweiten Hülsenteils (4, 6) ungefähr in einander entgegengesetzte Richtungen weisen und ungefähr in einer Ebene senkrecht zu der Längsachse liegen.

3. Trokarhülse nach Anspruch 2, **dadurch gekennzeichnet, dass** die zwei Endstellungen um genau 180° auseinander liegen und/oder dass in der Einführposition die beiden Flanschteile (14, 16) deckungsgleich übereinander liegen und/oder dass in der Arbeitsposition die beiden Flanschteile (14, 16) genau in einer Ebene senkrecht zu der Längsachse liegen.

4. Trokarhülse nach einem der vorhergehenden Ansprüche, **dadurch gekennzeichnet, dass** ein proximales Ende des ersten Hülsenteils (4) an einem Kopfteil (2) befestigt ist, der ggf. eine Dichtung (8) zum gasdichten Einführen eines Instruments in die Trokarhülse und einen Fluidanschlussstutzen (10) aufweist, wobei der Kopfteil (2) Führungs- und Verriegelungsmittel (22) enthält, in denen ein proximales Ende des zweiten Hülsenteils (6) für die Drehung um den ersten Hülsenteil (4) geführt ist und zumindest in der Endstellung des zweiten Hülsenteils (6), die der Arbeitsposition entspricht, verriegelbar ist.

5. Trokarhülse nach einem der vorhergehenden Ansprüche, **dadurch gekennzeichnet, dass** die Flanschteile (14, 16) einstückig mit dem jeweiligen Hülsenteil (4, 6) verbunden sind und/oder jeweils ungefähr 1 mm dick sind.

6. Trokarhülse nach einem der vorhergehenden Ansprüche, **dadurch gekennzeichnet, dass** die Flanschteile (14, 16) ungefähr U-förmige radiale Umrisse haben, wie in einer Ebene senkrecht zu der Längsachse gesehen, wobei der Abstand der beiden Schenkel der U-Form ungefähr gleich dem Durchmesser des ersten (4) oder vorzugsweise des zweiten Hülsenteils (6) ist.

7. Trokarhülse nach einem der vorhergehenden Ansprüche, **dadurch gekennzeichnet, dass** der am ersten Hülsenteil (4) anliegende Abschnitt des zweiten Hülsenteils (6) im Wesentlichen die Form eines geraden Rohrstücks hat.

8. Trokarhülse nach einem der Ansprüche 1 bis 6, **dadurch gekennzeichnet, dass** der am ersten Hülsenteil (4) anliegende Abschnitt des zweiten Hülsenteils (6) im Wesentlichen die Form einer Hälfte eines der Länge nach zweigeteilten Rohres hat.

9. Trokarhülse nach Anspruch 8, **dadurch gekennzeichnet, dass** der Querschnitt des zweiten Hülsenteils (6) senkrecht zu der Längsachse ein Kreisbogen ist, der einige Winkelgrade mehr als ein Halbkreis umfasst.

10. Trokarhülse nach Anspruch 1, **dadurch gekennzeichnet, dass** jeder der beiden Hülsenteile (34, 36) im Wesentlichen die Form eines geraden Voll-Rohres hat.

11. Trokarhülse nach Anspruch 10, **dadurch gekennzeichnet, dass** der erste Hülsenteil lösbar (34) am Kopfteil (32) befestigt ist und dass die beiden Hülsenteile (34, 36) derart ausgebildet sind, dass der zweite Hülsenteil (36) am ersten Hülsenteil (34) montiert und davon demontiert werden kann, wenn der erste Hülsenteil (34) vom Kopfteil (32) gelöst ist.

12. Trokarhülse nach Anspruch 11, **dadurch gekennzeichnet, dass** der zweite Hülsenteil (36) derart am ersten Hülsenteil (34) geführt ist, dass er zwischen zwei Endstellungen drehbar ist, die bezüglich der Längsachse um ungefähr 180° auseinander liegen, wobei die eine Endstellung einer Einführposition entspricht, in der die Flanschteile (44, 46) des ersten und des zweiten Hülsenteils (34, 36) im Wesentlichen in dieselbe Richtung weisen und genau in einer Ebene senkrecht zu der Längsachse liegen, und wobei die andere Endstellung einer Arbeitsposition entspricht, in der die Flanschteile (14, 16) des ersten und des zweiten Hülsenteils (4, 6) ungefähr in einander entgegengesetzte Richtungen weisen und ebenfalls genau in einer Ebene senkrecht zu der Längsachse liegen.

13. Trokarhülse nach Anspruch 12, **dadurch gekennzeichnet, dass** die Flanschteile (44, 46) des ersten und des zweiten Hülsenteils (34, 36) in der Einführposition zusammen einen U-förmigen radialen Umriss haben, wie in einer Ebene senkrecht zu der Längsachse gesehen.

14. Trokarhülse nach einem der Ansprüche 11 bis 14, **dadurch gekennzeichnet, dass** die lösbare Befestigung des erste Hülsenteils (34) am Kopfteil (32) einen Schraubverschluss (58, 60, 76, 78, 80) umfasst.

15. Trokarhülse nach einem der Ansprüche 11 bis 14, **dadurch gekennzeichnet, dass** die lösbare Befestigung des erste Hülsenteils (34') am Kopfteil (32') eine Art Bajonettverschluss (82, 84, 86) umfasst.

16. Trokar mit einer Trokarhülse nach einem der vorhergehenden Ansprüche und mit einem in den inneren Hülsenteil (34) passenden Trokardom (90), **dadurch gekennzeichnet, dass** der Trokardom (90) ein stumpfes distales Ende (92) hat, dessen Kontur mit der Kontur des distalen Endes der Trokarhülse im Wesentlichen bündig abschließt, wenn der Trokardom (90) vollständig in die Trokarhülse eingeführt ist.

17. Trokar mit einer Trokarhülse nach einem der vorhergehenden Ansprüche und mit einem Konterelement, das dafür eingerichtet ist, den ersten und den zweiten Hülsenteil (4, 6; 34; 36) ringförmig zu umschließen und die Flanschteile (14, 16; 44, 46) in Richtung der Längsachse zu kontern.

18. Trokar nach Anspruch 17, **dadurch gekennzeichnet, dass** das Konterelement eine einstückige geschlitzte Gummischeibe (26) ist.

19. Trokar nach Anspruch 17, **dadurch gekennzeichnet, dass** das Konterelement aus einem starren Element (102; 110) mit einem zum Außendurchmesser des äußeren Hülsenteils (6, 36) passenden Schlitz (112) und einem von dem starren Element (102; 110) lösbaren elastischen Element (100; 114) besteht.

20. Trokar nach Anspruch 17, **dadurch gekennzeichnet, dass** das Konterelement nur aus starren Elementen besteht, nämlich aus einer Platte (120) mit einem zum Außendurchmesser des äußeren Hülsenteils (6, 36) passenden Schlitz (122), einem auf der Platte (120) verschiebbaren Klemmelement (124) für den äußeren Hülsenteil (6, 36) und einem auf der Platte (120) gelagerten Betätigungselement (126, 128) für das verschiebbare Klemmelement (124).

## Claims

1. Trocar sheath for minimally invasive surgery, with a first sheath part (4; 34), which has substantially the shape of a straight pipe section with a longitudinal axis, and with a second sheath part (6; 36), which bears tightly on and at least partially surrounds the first sheath part (4) and, during use, is movable relative to the first sheath part (4), **characterized in that** the mobility of the second sheath part (6; 36) relative to the first sheath part (4) lies substantially only in a rotatability of the second sheath part (6; 36) about the first sheath part (4; 34), and **in that** the first sheath part (4; 34) and the second sheath part (6; 36) each have, at their distal axial ends, a flange part (14, 16; 44, 46) which extends radially outwards from the respective sheath part (4, 6; 34, 36) about an angle of less than 180°.

2. Trocar sheath according to Claim 1, **characterized in that** the second sheath part (6) is guided in such a way that it is rotatable between two end positions which lie approximately 180° from each other with respect to the longitudinal axis, wherein one end position corresponds to an insertion position, in which the flange parts (14, 16) of the first and second sheath part (4, 6) point in the same direction and lie substantially one over the other, and wherein the other end position corresponds to a working position, in which the flange parts (14, 16) of the first and second sheath part (4, 6) point approximately in mutually opposite directions and lie approximately in a plane perpendicular to the longitudinal axis.

3. Trocar sheath according to Claim 2, **characterized in that** the two end positions lie exactly 180° from each other, and/or **in that** the two flange parts (14, 16) lie congruently one over the other in the insertion position, and/or **in that** the two flange parts (14, 16) lie exactly in a plane perpendicular to the longitudinal axis in the working position.

4. Trocar sheath according to one of the preceding claims, **characterized in that** a proximal end of the first sheath part (4) is fastened to a head part (2) which, if appropriate, has a seal (8), for gas-tight insertion of an instrument into the trocar sheath, and a fluid connector piece (10), wherein the head part (2) contains guiding and locking means (22) in which a proximal end of the second sheath part (6) is guided for the rotation about the first sheath part (4) and which can be locked, at least in the end position of the second sheath part (6) corresponding to the working position.

5. Trocar sheath according to one of the preceding claims, **characterized in that** the flange parts (14, 16) are integrally connected to the respective sheath part (4, 6) and/or are each approximately 1 mm thick.

6. Trocar sheath according to one of the preceding claims, **characterized in that** the flange parts (14, 16) have approximately U-shaped radial contours, as seen in a plane perpendicular to the longitudinal axis, wherein the distance between the two legs of the U shape is approximately equal to the diameter of the first sheath part (4) or preferably of the second sheath part (6).

7. Trocar sheath according to one of the preceding claims, **characterized in that** the portion of the second sheath part (6) bearing on the first sheath part (4) has substantially the shape of a straight pipe section.

8. Trocar sheath according to one of Claims 1 to 6, **characterized in that** the portion of the second sheath part (6) bearing on the first sheath part (4) has substantially the shape of half of a pipe that is divided in two lengthwise.

9. Trocar sheath according to Claim 8, **characterized in that** the cross section of the second sheath part (6) perpendicular to the longitudinal axis is an arc of a circle, which comprises several angle degrees more than a semicircle.

10. Trocar sheath according to Claim 1, **characterized in that** each of the two sheath parts (34, 36) has substantially the shape of a straight solid pipe.

11. Trocar sheath according to Claim 10, **characterized in that** the first sheath part (34) is fastened releasably to the head part (32), and **in that** the two sheath parts (34, 36) are designed in such a way that the second sheath part (36) can be mounted on the first sheath part (34) and removed therefrom when the first sheath part (34) is released from the head part (32).

12. Trocar sheath according to Claim 11, **characterized in that** the second sheath part (36) is guided on the first sheath part (34) in such a way that it is rotatable between two end positions which lie approximately 180° from each other with respect to the longitudinal axis, wherein one end position corresponds to an insertion position, in which the flange parts (44, 46) of the first and second sheath part (34, 36) point substantially in the same direction and lie exactly in a plane perpendicular to the longitudinal axis, and wherein the other end position corresponds to a working position, in which the flange parts (14, 16) of the first and second sheath part (4, 6) point approximately in mutually opposite directions and likewise lie exactly in a plane perpendicular to the longitudinal axis.

13. Trocar sheath according to Claim 12, **characterized in that** the flange parts (44, 46) of the first and second sheath part (34, 36) together have a U-shaped radial contour in the insertion position, as seen in a plane perpendicular to the longitudinal axis.

14. Trocar sheath according to one of Claims 11 to 14, **characterized in that** the releasable fastening of the first sheath part (34) to the head part (32) comprises a screw fastener (58, 60, 76, 78, 80).

15. Trocar sheath according to one of Claims 11 to 14, **characterized in that** the releasable fastening of the first sheath part (34') to the head part (32') comprises a kind of bayonet fastener (82, 84, 86).

16. Trocar with a trocar sheath according to one of the preceding claims and with a trocar mandrel (90) fitting into the inner sheath part (34), **characterized in that** the trocar mandrel (90) has a blunt distal end (92), of which the contour terminates substantially flush with the contour of the distal end of the trocar sheath when the trocar mandrel (90) is inserted fully into the trocar sheath.

17. Trocar with a trocar sheath according to one of the preceding claims and with a locking element which is designed to enclose the first and second sheath part (4, 6; 34, 36) in a ring shape and to lock the flange parts (14, 16; 44, 46) in the direction of the longitudinal axis.

18. Trocar according to Claim 17, **characterized in that** the locking element is a one-piece slit rubber disc (26).

19. Trocar according to Claim 17, **characterized in that** the locking element is composed of a rigid element (102; 110), with a slit (112) matching the external diameter of the outer sheath part (6, 36), and of an elastic element (100; 114) releasable from the rigid element (102; 110).

20. Trocar according to Claim 17, **characterized in that** the locking element is composed only of rigid elements, namely a plate (120) with a slit (122) matching the external diameter of the outer sheath part (6, 36), a clamping element (124) movable on the plate (120) and provided for the outer sheath part (6, 36), and an actuating element (126, 128) mounted on the plate (120) and provided for the movable clamping element (124).

## Revendications

1. Gaine de trocart pour chirurgie minimalement invasive, présentant
une première partie de gaine (4; 34) qui présente essentiellement la forme d'une pièce tubulaire rectiligne dotée d'un axe longitudinal et
une deuxième partie de gaine (6; 36) qui entoure la première partie de gaine (4) et apte à se déplacer par rapport à la première partie de gaine (4) en utilisation,
**caractérisée en ce que**
la mobilité de la deuxième partie de gaine (6; 36) par rapport la première partie de gaine (4) n'est essentiellement constituée que d'une possibilité de rotation de la deuxième partie de gaine (6; 36) autour de la première partie de gaine (4; 34) et
**en ce que** la première partie de gaine (4; 34) et la deuxième partie de gaine (6; 36) présentent chacune à leur extrémité axiale distale une partie en bride (14, 16; 44; 46) qui s'étend sur un angle inférieur à 180° radialement vers l'extérieur de la partie de gaine (4, 6; 34, 36) concernée.

2. Gaine de trocart selon la revendication 1, **caractérisée en ce que** la deuxième partie de gaine (6) est guidée de manière à pouvoir tourner entre deux positions d'extrémité qui sont situées à environ 180° l'une de l'autre par rapport à l'axe longitudinal, une des positions d'extrémité correspondant à une position d'insertion dans laquelle les parties de bride (14, 16) de la première et de la deuxième partie de gaine (4, 6) sont orientées dans la même direction et sont situées essentiellement l'une au-dessus de l'autre, l'autre position d'extrémité correspondant à une position de travail dans laquelle les parties de bride (14, 16) de la première ou de la deuxième partie de gaine (4, 6) sont orientées sensiblement dans des directions mutuellement opposées et sont situées sensiblement dans un plan perpendiculaire à l'axe longitudinal.

3. Gaine de trocart selon la revendication 2, **caractérisée en ce que** les deux positions d'extrémité sont situées exactement à 180° l'une de l'autre et/ou en ce que dans la position d'insertion, les deux parties de bride (14, 16) sont situées l'une au-dessus de l'autre et se recouvrent et/ou **en ce que** dans la position de travail, les deux parties de bride (14, 16) sont situées exactement dans un plan perpendiculaire à l'axe longitudinal.

4. Gaine de trocart selon l'une des revendications précédentes, **caractérisée en ce qu'**une extrémité proximale de la première partie de gaine (4) est fixée sur une partie de tête (2) qui présente éventuellement un joint d'étanchéité (8) qui permet d'insérer un instrument de manière étanche aux gaz dans la gaine de trocart et une tubulure (10) de raccordement de fluide, la partie de tête (2) contenant des moyens de guidage et de verrouillage (22) dans lesquels une extrémité proximale de la deuxième partie de gaine (6) est guidée de manière à pouvoir tourner autour de la première partie de gaine (4) et peut être verrouillée au moins dans la position d'extrémité de la deuxième partie de gaine (6) qui correspond à la position de travail.

5. Gaine de trocart selon l'une des revendications précédentes, **caractérisée en ce que** les parties de bride (14, 16) sont raccordées d'un seul tenant à chaque partie de gaine (4, 6) et/ou ont chacune une épaisseur d'environ 1 mm.

6. Gaine de trocart selon l'une des revendications précédentes, **caractérisée en ce que** les parties de bride (14, 16) présentent des rainures périphériques radiales sensiblement en forme de U dans un plan perpendiculaire à l'axe longitudinal, la distance entre les deux branches de la forme du U étant sensiblement égale au diamètre de la première partie de gaine (4) ou de préférence de la deuxième partie de gaine (6).

7. Gaine de trocart selon l'une des revendications précédentes, **caractérisée en ce que** la section de la deuxième partie de gaine (6) qui repose contre la première partie de gaine (4) présente essentiellement la forme d'une pièce tubulaire rectiligne.

8. Gaine de trocart selon l'une des revendications 1 à 6, **caractérisée en ce que** la section de la deuxième partie de gaine (6) qui repose contre la première partie de gaine (4) présente essentiellement la forme de la moitié d'un tube divisé en deux dans le sens de sa longueur.

9. Gaine de trocart selon la revendication 8, **caractérisée en ce que** la section transversale de la deuxième partie de gaine (6) perpendiculairement à l'axe longitudinal est un arc de cercle qui comporte quelques degrés de plus qu'un demi-cercle.

10. Gaine de trocart selon la revendication 1, **caractérisée en ce que** chacune des deux parties de gaine (34, 36) présente essentiellement la forme d'un tube plein rectiligne.

11. Gaine de trocart selon la revendication 10, **caractérisée en ce que** la première partie de gaine (34) est fixée de manière libérable sur la partie de tête (32) et **en ce que** les deux parties de gaine (34, 36) sont configurées de telle sorte que la deuxième partie de gaine (36) soit montée sur la première partie de gaine (34) et puisse en être démontée lorsque la première partie de gaine (34) est libérée de la partie de tête (32).

12. Gaine de trocart selon la revendication 11, **caractérisée en ce que** la deuxième partie de gaine (36) est guidée sur la première partie de gaine (34) de manière à pouvoir tourner entre deux positions d'extrémité qui sont situées à environ 180° l'une de l'autre par rapport à l'axe longitudinal, l'une des positions d'extrémité correspondant à une position d'insertion dans laquelle les parties de bride (44, 46) de la première ou de la deuxième partie de gaine (34, 36) sont orientées essentiellement dans la même direction et sont situées exactement dans un plan perpendiculaire à l'axe longitudinal, l'autre position d'extrémité correspondant à une position de travail dans laquelle les parties de bride (14, 16) de la première et de la deuxième partie de gaine (4, 6) sont orientées sensiblement dans des directions mutuellement opposées et sont également situées exactement dans un plan perpendiculaire à l'axe longitudinal.

13. Gaine de trocart selon la revendication 12, **caractérisée en ce que** les parties de bride (44, 46) de la première et de la deuxième partie de gaine (34, 36) présentent ensemble dans la position d'insertion un périmètre radial en forme de U, par exemple dans un plan perpendiculaire à l'axe longitudinal.

14. Gaine de trocart selon l'une des revendications 11 à 14, **caractérisée en ce que** la fixation libérable de la première partie de gaine (34) sur la partie de tête (32) comporte un raccord fileté (58, 60, 76, 78, 80).

15. Gaine de trocart selon l'une des revendications 11 à 14, **caractérisée en ce que** la fixation libérable de la première partie de gaine (34') sur la partie de tête (32') comporte une fermeture (82, 84, 86) de type à baïonnette.

16. Trocart doté d'une gaine de trocart selon l'une des revendications précédentes et d'un mandrin de trocart (90) qui s'adapte dans la partie intérieure de gaine (34), **caractérisé en ce que** le mandrin de trocart (90) présente une extrémité distale émoussée (92) dont le contour ferme essentiellement à chant le contour de l'extrémité distale de la gaine de trocart lorsque le mandrin de trocart (90) est inséré complètement dans la gaine de trocart.

17. Trocart doté d'une gaine de trocart selon l'une des revendications précédentes et d'un élément complémentaire conçu pour enfermer en anneau la première et la deuxième partie de gaine (4, 6; 34; 36) et contrer les parties de bride (14, 16; 44, 46) dans la direction de l'axe longitudinal.

18. Trocart selon la revendication 17, **caractérisé en ce que** l'élément complémentaire est un disque en caoutchouc (26) fendu et d'un seul tenant.

19. Trocart selon la revendication 17, **caractérisé en ce que** l'élément complémentaire est constitué d'un élément rigide (102; 110) doté d'une fente (112) qui s'adapte au diamètre extérieur de la partie extérieure de gaine (6, 36) et d'un élément élastique (100; 114) libérable de l'élément rigide (102; 110).

20. Trocart selon la revendication 17, **caractérisé en ce que** l'élément complémentaire est constitué uniquement d'un élément rigide, à savoir d'une plaque (120) dotée d'une fente (122) qui s'adapte au diamètre extérieur de la partie extérieure de gaine (6, 36), d'un élément de serrage (124) apte à coulisser sur la plaque (120) pour la partie extérieure de gaine (6, 36) et d'un élément d'actionnement (126, 128) de l'élément de serrage coulissant (124) monté sur la plaque (120).
